# EUROPEAN PATENT SPECIFICATION

(11) **EP 3 838 230 B1**
(45) Date of publication and mention of the grant of the patent: **27.08.2025**
(21) Application number: 19216468.9
(22) Date of filing: 16.12.2019
(51) Int. Cl.: A61F 2/40

(54) **ANCHORING MEMBER FOR A JOINT REPLACEMENT**
VERANKERUNGSELEMENT FÜR EINEN GELENKERSATZ
ÉLÉMENT D'ANCRAGE DE REMPLACEMENT DE JOINTS

(43) Date of publication of application: 23.06.2021
(60) Divisional application: 25190123.7
(73) Proprietor: Waldemar Link GmbH & Co. KG, 22339 Hamburg (DE)
(72) Inventor: DALLA PRIA, Paolo, 33100 Udine (IT); SCHULTZ, Thomas, 21365 Adendorf (DE)
(74) Representative: Alatis

(56) References cited:
- EP-A1- 0 581 667
- WO-A1-2019/178104
- GB-A- 2 443 163
- US-A- 5 702 457
- US-A1- 2005 261 775
- US-A1- 2008 294 268

## Description

### FIELD OF THE INVENTION

The present invention generally relates to joint replacements and in particular to shoulder joint replacements. More particularly, the present invention relates to an anchoring member that can be anchored within bone tissue.

### BACKGROUND

A native joint may undergo degenerative changes for a variety of reasons, for instance arthritis. Also, a joint may be fractured or otherwise be damaged by an external force affecting the functionality of its joint surfaces, in particular the hyaline cartilage forming these surfaces. If such a joint is significantly degenerated or damaged, it may become necessary to replace the native joint with a joint replacement.

Such a replacement may begin with a surface replacement and/or a hemi-arthroplasty, both being a partial replacement of a native synovial joint. This is generally only a temporary solution that will likely be followed by total joint replacement. Further, the joint replacement is also subject to wear and may have to be replaced, a so-called revision.

For example, a total shoulder replacement comprises a humeral component and a glenoid component. However, for a hemi-arthroplasty, the humeral component articulates against the native glenoid cavity.

Typically, the humeral component comprises an anchoring member for insertion into the humerus and a joint member that provides a joint surface which may be a convex head in case of an anatomic arthroplasty or a concave cup in case of reverse arthroplasty. Typically, the joint member is coupled to a neck portion of the anchoring member at an inclined angle to the longitudinal axis of a distal portion of the anchoring member, i. e. the longitudinal axis of the humerus, in order to recreate the arrangement of the natural joint.

In an anatomic arthroplasty, the structure of a joint replacement resembles an anatomical shoulder with a convex anatomic ball head fixed to the humerus and a concave anatomic glenoid component fixed to the scapula.

In a reverse arthroplasty, the anatomic configuration is reversed, i. e. a reverse concave liner is fixed to the humerus and a reverse convex glenosphere is fixed to the scapula.

Similar to the knee joint, the shoulder joint is primarily stabilized by the ligaments and muscles surrounding this joint. Thus, in any of the surgical techniques mentioned above it is important to keep as much soft tissue intact as possible for the stability of the treated joint. In particular the shoulder joint is prone to luxation and even more so after surgery.

Further, the following problems have been observed with joint replacements and in particular with shoulder joint replacements. When anchoring a joint replacement within a bone, the joint member may require a larger volume than the native joint. This may result in the joint member compressing the soft tissue surrounding this joint (muscles, tendons, ligaments, nerves, vasculature) since the soft tissue is natively dimensioned for the native joint member.

In case of shoulder joint replacements, this problem primarily affects the soft tissue cranially of the joint, which is then impinged, for example between the humerus head and the (native) acromion. This leads to overstretched muscles and nerves in the vicinity of the joint causing pain, and, in the case of joint replacements, often in a limitation of abductive motion of the respective arm. This problem is known as "overstuffing".

EP 1 978 896 B1 describes a humeral component of a shoulder joint prosthesis, with an anchoring member and a joint member, in which the anchoring member and the joint member are connected to each other via a flat interface between the anchoring member and the joint member. The joint member is placed on top of the anchoring member, so that a large volume is required for the joint replacement.

EP 1 064 890 B1 is directed to a glenosphere for a shoulder joint replacement. This document discloses a reverse shoulder joint replacement in which the glenosphere interacts with a joint member that is connected to an anchoring member, wherein the connection between the anchoring member and the joint member is formed in a flat shape and located inside the humerus.

Further joint replacements that are configured to be attached to bone tissue are disclosed in US 5 702 457 A, US 2008/294268 A1, EP 0 581 667 A1, US 2005/261775 A1, WO 2019/178104 A1 and GB 2 443 163 A.

### SUMMARY OF THE INVENTION

In view of the above, it is therefore an objective of the present disclosure to provide a shoulder joint replacement that allows for a better adaptation in order to prevent overstuffing or understuffing of soft tissue surrounding the shoulder joint.

This objective is addressed by an anchoring member for a joint replacement according to independent claim 1, wherein the claims dependent thereon relate to preferred embodiments.

More specifically, the present disclosure provides an anchoring member for a joint replacement, wherein the anchoring member comprises a proximal interface part and a distal portion. The distal portion is configured to be anchored within bone tissue and the proximal interface part is configured for mounting a joint member of the joint replacement. The proximal interface part comprises a concave interface surface.

An anchoring member for a joint replacement serves the purpose of anchoring the joint replacement in a bone. For example, in case of a shoulder joint replacement, the anchoring member is to be implanted into a cavity prepared at the proximal end of a humerus. If the joint replacement is a hip or wrist joint replacement, the anchoring member is, for example, inserted into the femur or into the radius, respectively. Consequently, the anchoring member can be inserted into any long bone for implanting a joint replacement member.

The anchoring member is configured for attaching a joint member via the proximal interface part, which joint member has the purpose of forming a joint with a corresponding joint member of the joint counterpart.

The proximal interface part of the anchoring member comprising a concave interface surface has the advantage that the joint member can be mounted more distal or deeper into the anchoring member, thus reducing the space of the joint replacement. Particularly in case of a reverse shoulder joint replacement, this effect is particularly beneficial for providing the desired functionality and range of motion.

Also, such a concavity reduces the distance between the humerus and the glenoid. When using a joint member (e. g. a reverse liner for a reverse joint replacement) such a joint member has a defined thickness and interacts with a convex hemisphere ("glenosphere") connected to the glenoid. Therefore, the concavity in the anchoring member can be coupled to a convex counterpart of the joint member and due to this convexity, the thickness of the reverse joint member can be reduced in comparison to, for example, a flat surface interface.

Further, this configuration of the proximal interface part reduces the protrusion of the joint member in the axial direction such that the impingement of tissue surrounding the joint, such as ligaments, muscles, and tendons, is less likely.

The concave interface surface of the anchoring member comprises an indexing structure.

Providing such an indexing structure for the anchoring member allows to adjust and fixate the relative orientations of the joint member in relation to the anchoring member in cooperation with a corresponding complementary indexing structure at the joint member. In the case of a shoulder joint replacement, this feature allows such an adjustment for the relative orientation between the joint member and the humerus.

Since different patient anatomies are likely to require different replacements or implants in order to restore the functionality of the native joint, it is preferred to be able to select between different orientations between the joint member and the anchoring member. Providing an anchoring member with an indexing structure allows to implement different joint replacement configurations using the same anchoring member and joint member. In other words, this allows for adjusting the orientation about an axis between the anchoring member and the joint member. This reduces the number of parts of a joint replacement that are required to be kept on stock for a joint replacement surgery.

The concave interface surface may be formed as a two-dimensional or three-dimensional concavity. A two-dimensional concavity has a concave profile in one plane (and in all planes parallel thereto), whereas in a plane perpendicular to this plane, the profile of the two-dimensional concavity is not concave. Instead, it is preferably linear but may also be convex. The plane comprising the concave profile preferably extends in a proximal distal direction of the joint replacement. Further, the profile is preferably a curved profile and even more preferred a circular or elliptical profile.

However, the concave interface surface is preferably formed as a three-dimensional concavity. Such a concave interface surface is formed as a recess. As a result, this concavity has a concave profile in one plane and in another plane perpendicular thereto or at any other angle in between.

Thus, the term "recess" is to be understood in this disclosure as an indentation, i.e. the surface of the recess has a point or section that is deeper than an annular section and is completely surrounded by this annular section. In other words, the interface surface bulges inwards.

Preferably, the concave recess of the anchoring member has a curved profile, the curved profile in particular being spherical, ellipsoidal, or ovoid shaped.

Such a profile is three-dimensionally curved, which is to be understood herein as being curved in at least two non-identical and non-parallel planes such that a spherical, ellipsoidal, ovoid, or otherwise three-dimensionally curved shape is formed.

In contrast to alternative shapes, such as flat shapes or shapes that are curved in only one plane, this three-dimensionally curved shape of the concave recess provides the advantage that the relative orientation between the anchoring member and the joint member can be adjusted about two axes.

The anchoring member may comprise a mounting hole for fixedly attaching a joint member and the mounting hole preferably comprises a thread for engagement with a fixation member and/or a tapered portion.

A mounting hole between the anchoring member and the joint member provides a rotational axis for the fine adjustment of the relative orientations of the joint member and the anchoring member. In case of a shoulder joint, this results in an adjustment of the relative orientation of the joint member and the humerus bone.

Preferably, the anchoring member comprises a plurality of fins extending in radial directions in relation to a predefined direction of insertion of the anchoring member into the bone.

Providing these fins has the advantage of locking the anchoring member in a rotational direction around the axis of insertion of the anchoring member. Further, these fins provide additional surface area for anchoring within bone tissue. This additional surface also enables to provide the anchoring member with a more compact design. In other words, providing the anchoring member with fins allows for reducing the size of the distal portion of the anchoring member.

More generally, such a configuration may be considered as a substantially stemless configuration since it does not comprise a longitudinal stem, e. g. a stem that is configured to extend into the medullary channel of a long bone. In particular, such a stemless configuration has preferably a length to width ratio of less than 1.5 and even more preferably 1. Accordingly, other protruding anchoring structures than fins may be used.

Preferably, the indexing structure of the anchoring member comprises at least one indexing recess, in particular a plurality of indexing recesses, the at least one indexing recess being for mating with a corresponding protrusion of the joint member. Even more preferably, this indexing structure is located around the mounting hole of the anchoring member.

When the anchoring member and the joint member are connected by engaging the mounting hole of the anchoring member and a corresponding part of the joint member, it is generally possible that the relative orientations of the joint member and the anchoring member can be changed by rotating the joint member relatively to the anchoring member around an axis of rotation through the mounting hole.

Including at least one indexing recess that is provided in a distance to an axis of rotation, wherein the axis of rotation corresponds to the longitudinal axis of the mounting hole, allows for fixing the relative position of the anchoring member and the joint member by a form fit. Naturally, such an indexing structure may also form a part of the anchoring member's mounting hole and the corresponding protrusion of the joint member.

Providing more than one indexing recess further enables a selection between different options of arranging the relative orientation of the anchoring member and the joint member, which in turn allows for different patient anatomies to be treated with a fewer number of joint members and anchoring members that need to be prepared for a single joint replacement procedure.

Preferably, the anchoring member further comprises a stem part that is configured to be inserted into the bone, wherein the stem part is even more preferably modular.

Since the stability of the fixation of the anchoring part within a bone of a patient, into which the anchoring part is inserted, is largely determined by the interaction between the bone and the anchoring member, a stem part for insertion into the bone increases stability.

A modular stem part provides on the one hand the possibility of using different combinations of materials or surface textures for different sections of the stem part without the need for keeping each combination in stock. For example, one material and/or texture is provided for the portion of the anchoring member to be positioned in the medullary cavity and another material and/or texture is provided for the portion to be positioned in the trabecular portion of the bone. Yet another material and/or texture may be provided for the portion that is to be positioned, where muscle or soft tissue is present.

Preferably, the joint member comprises a proximal end and a distal interface part, the proximal end being formed as a joint surface and the distal interface part being configured for mounting an anchoring member of the joint replacement, in particular an anchoring member as described above. The joint surface is a ball joint surface and the distal interface part comprises a convex interface surface.

It is to be noted that the term "ball joint surface" as used herein can be used for both the surface of the ball portion of the ball joint, and for the cavity portion of the ball joint.

The ball joint surface may, for example, be a surface that resembles the configuration of a native joint. In case of a shoulder joint replacement, the joint surface of a humeral joint replacement can for example resemble the configuration of a native humerus head that mates with a corresponding glenoid cavity.

In case of a reverse joint replacement, the joint surface forms a cavity to be arranged at the side of the humerus component of the shoulder joint replacement, which is configured to form a joint with a corresponding ball-shaped surface of the joint replacement attached to the glenoid bone.

In both cases, the distal interface part of the joint member is convex, which - similarly to the discussion above - is curved along two non-identical and non-parallel axes.

This configuration allows for the distal interface part of the joint member to engage with a complementary proximal interface part of an anchoring member.

The connection between the anchoring member and the joint member is particularly preferred when the proximal interface part of the anchoring member and the distal interface part of the joint member have portions with basically identical radii of curvatures.

On the one hand, if in contact when being connected, the surface contact resulting from this configuration provides a stable support for the joint member. This also reduces gaps between the anchoring member and the joint member. Avoiding such gaps reduces dead spaces and, thus, prevents fibrous tissue to be formed therein and the ingress of germs.

On the other hand, this configuration is kept from interfering with each other if the connection between the anchoring member and the joint member is established by a tapered connection between the mounting hole and a corresponding protrusion of the joint member. In order to ensure a reliable friction fit of the tapered connection, there should be not contact between the concave surface of the interface part at least partly surrounding the mounting hole and the convex surface of the interface part at least partly surrounding aforementioned corresponding protrusion. The two interface surfaces having basically the same radii ensures a minimum distance between them in an assembled state besides all the other advantage previously discussed.

Preferably, the joint member comprises an intermediate component and a joint component, wherein the intermediate component and the joint component are preferably fixedly attached to each other by means of a friction fit or a form fit.

Providing an intermediate component and a joint component for the joint member has, for example, the advantage that an individual intermediate component can be appropriately chosen among a plurality of different intermediate components in order to adapt the joint replacement to a patient's particular anatomy. If at all, the joint component may then only differ in the dimensions of its joint surface.

Generally, the components can be affixed to each other by any method known in the art, however, a friction fit and a form fit have proven to provide a secure connection.

For example, the intermediate component can be a concentric or eccentric adapter, and the joint component can be an anatomic head that resembles the surface of the humerus head.

In case of a reverse joint replacement, the intermediate component may, for example, be a neutral, offset or inclined tray, or an adapter or an extender, while the joint component can be a reverse insert, for example a neutral reverse insert or a reverse insert with an inclination of, for example between 5° to 25°.

Inclined configurations and offsets of the intermediate component and joint component allow the surgeon to choose the most appropriate configuration for every specific case. This is particularly the case if each of the intermediate component and the joint component are provided with different inclinations. This allows to adjust the angle between the stem and the central axis of the joint component's concave joint surface as desired. Further, it is possible to correct or adjust the orientation of the articular joint in different planes by the choice of the inclined intermediate component and joint component.

While historically, the angle between the axis of the stem and a plane of the joint component has been set to approximately 155° for reasons of joint stability, nowadays, an angle of approximately 135° is considered safe. The lower the angle, the lower the risk for impingement between the humeral implant and the scapular bone. Providing a joint replacement with an angle of, for example, 135° instead of 155° also generally resembles the native anatomy better, provides advantages regarding the surgical procedure and facilitates the conversion from an anatomical configuration to a reverse configuration.

Alternatively or additionally, the adaptation of the angle between the longitudinal axis of the stem and the central axis of the concave joint surface may be adjusted if an indexing structure is provided. Due to the inclination of the intermediate component, adjusting the orientation of the intermediate component in relation to the anchoring member adapts the inclination in the frontal plane of a patient.

The inserts may, for example, be made of materials like CoCr or Polyethylene (PE) such as UHMWPE. They may have a coating.

Further, integrating an intermediate component in the joint replacement's configuration allows switching between the anatomic configuration and the reverse configuration of the replacement minimizing or even preventing an effect on the fixation of the joint replacement to bone tissue and/or soft tissue.

Usually, using such inserts would lead to an increase in space required for the joint replacement. However, when using the concave proximal interface surface, the wall thickness of the insert may be lowered without any risk of instability or material failure. Also, the risk of overstuffing and overstretching the soft tissue surrounding the joint replacement can be at least reduced, resulting in the desired function and desired range of motion of the joint replacement. This results in another increase in quality of life for the patient.

The intermediate component and the joint component may be attached to each other by a friction fit, for example by a morse type taper or by a threaded connection. Alternatively or additionally, the components may be attached to each other by a form fit, for example a snap fit or a bayonet connection.

Preferably, the interface surface of the joint member includes an indexing structure, the indexing structure even more preferably being a protrusion configured so as to mate with an indexing recess of an anchoring member.

As described above, such an indexing structure provides the option to fixate and/or adjust the relative orientation of the anchoring member and the joint member in order to accommodate for different patient anatomies.

The joint member may be configured as an anatomical or reverse joint member. This allows for an engagement with a corresponding anatomic glenoid replacement or a reverse glenosphere replacement.

It is also disclosed a joint replacement component for replacing one side of a joint. The joint replacement component comprises an anchoring member and a joint member having any of the features described above. In an assembled state, the concave interface surface of the anchoring member and the convex interface surface of the joint member cause the joint member to be partly accommodated within the anchoring member.

This assembly allows to reduce the space required for the joint replacement component since a portion of the joint member is accommodated within the concavity or recess of the anchoring member formed by the concave interface surface.

Further, this configuration provides an enhanced stiffness of the assembled joint replacement due to a more compact build of the anchoring member.

In case that the connection between the anchoring member and the joint member is a taper connection, it is desirable that the proximal surface of the anchoring member and the distal surface of the joint member do not contact each other. Still, the concave interface surface allows for a reduced space needed by the joint replacement component.

Preferably, the joint replacement is a replacement for a spherical joint, in particular for a shoulder joint.

Applying the present disclosure to shoulder joints is particularly beneficial since shoulder joint replacements are particularly prone to space limitations that increase the likelihood that a problem due to overstuffing occurs. The configuration of the anchoring member as disclosed herein supports the desired outcome for a patient subject to such a joint replacement.

### BRIEF DESCRIPTION OF THE DRAWINGS

- Fig. 1 shows an anchoring member for a joint replacement according to an embodiment.
- Figs. 2A, 2B , and 2C show another anchoring member for a joint replacement according to another embodiment.
- Fig. 3 shows, in a side view, an arrangement of a joint replacement component.
- Figs. 4A, 4B, and 4C show different views of an example of the distal interface part.
- Fig. 5A shows an embodiment of a modular anchoring portion.
- Fig. 5B shows an embodiment of a proximal part of the anchoring portion.
- Fig. 6 shows exploded views of different examples of a reverse joint replacement.
- Figs. 7A and 7B show an example of an intermediate component.
- Fig. 8 shows an example of a reverse joint component.
- Figs. 9A and 9B show an embodiment of an intermediate component.
- Figs. 10A and 10B show an example of a reverse joint component.
- Fig. 11 shows reverse joint components according to further examples.

### DETAILLED DESCRIPTION OF PREFERRED EMBODIMENTS

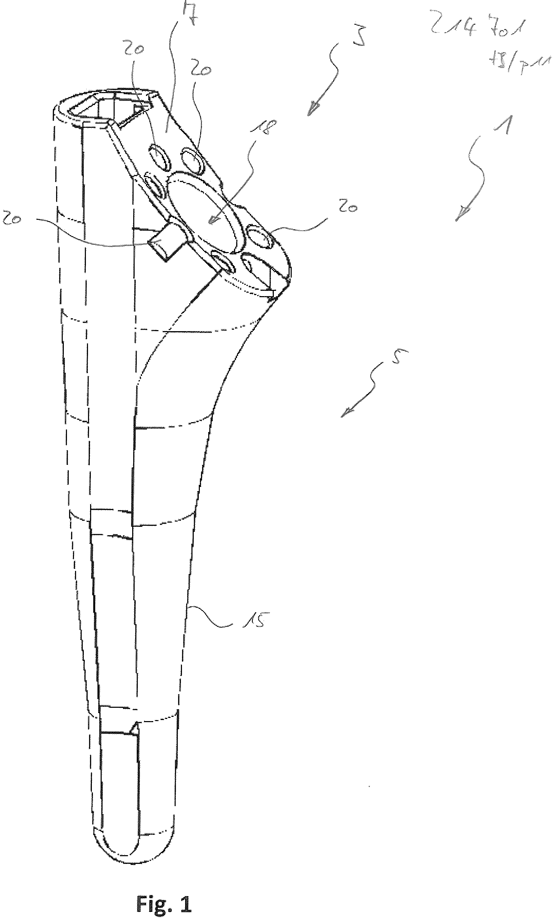

Fig. 1 shows an anchoring member 1 for a joint replacement according to an embodiment.

The anchoring member 1 has two portions, namely, a proximal interface part 3, and a distal portion 5. The distal portion 5 of the embodiment shown in Fig. 1 has a stem part 15. In this embodiment, stem part 15 has an elongate shape and is configured so as to be insertable into a long bone, for example, into a humerus or a femur, so that the distal portion 5 can be anchored within the bone tissue of such a bone.

The proximal interface part 3 is configured for mounting a joint member of the joint replacement and comprises a concave interface surface 7. As illustrated, the concave interface surface 7 may be formed as a recess, i. e. as an inwardly bulging indentation. Nonetheless, instead of being formed as a three-dimensional concavity, it may also be formed as a two-dimensional concavity as described above.

The proximal interface part 3 of the embodiment shown in Fig. 1 further comprises a central hole 18, and a plurality of indexing recesses 20 arranged around the central hole 18. In this embodiment, the indexing recesses 20 are arranged equidistantly on a circle around the central axis of the mounting hole 18. The mounting hole 18 can be provided with a thread (not shown). Alternatively, the mounting hole 18 can be configured as a tapered hole.

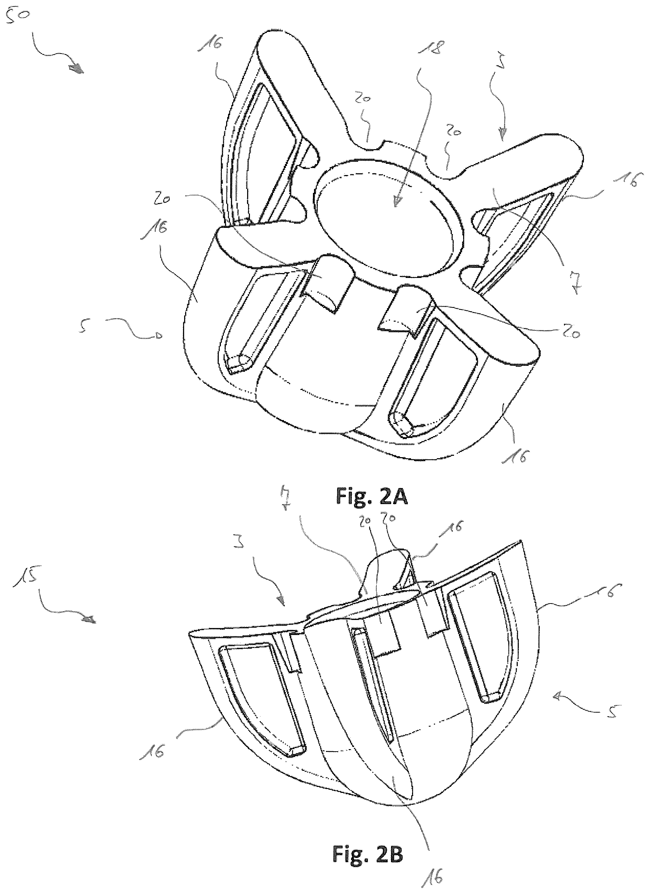

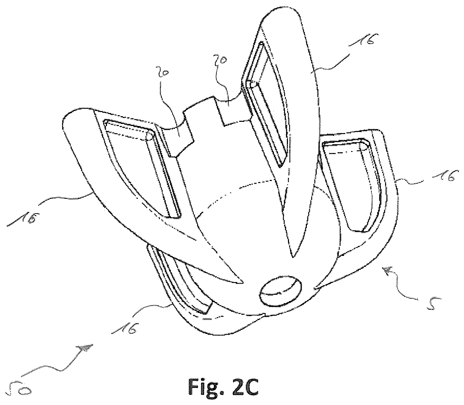

Figs. 2A, 2B , and 2C show another anchoring member 50 for a joint replacement according to another embodiment in different views. The embodiment shown in Figs. 2A, 2B , and 2C shows an anchoring member 50, which similarly to the anchoring member 1 shown in Fig. 1 has two portions, namely a proximal interface part 3 and a distal portion 5.

The proximal interface part 3 is configured for mounting a joint member of the joint replacement and comprises a concave interface surface 7. Similar to the embodiment shown in Fig. 1 , the concave interface surface 7 is also preferably formed as a three-dimensional recess.

The proximal interface part 3 of the embodiment shown in Fig. 2 further comprises a central hole 18. As illustrated, a plurality of indexing recesses 20 may be arranged equidistantly around the mounting hole 18 (cf. Fig. 1 ).

The distal portion 5 of the anchoring member 50 is configured to be inserted into a long bone, such as a humerus. The direction of insertion defines an axis of insertion of the anchoring member 50, which in case of the anchoring member 50 shown in Fig. 2 , is the central axis of the mounting hole 18. However, the axis of insertion is not limited to correspond to the axis of the mounting hole 18 but can be different therefrom.

In the embodiment shown in Figs. 2A, 2B , and 2C , the distal portion 5 comprises a plurality of fins 16 that extend in radial directions in relation to the insertion direction of the anchoring member 50. The embodiment shown comprises four fins 16, and pairs of two essentially symmetrical fins 16 are arranged opposite to each other with respect to the axis of insertion into the bone. It is to be noted that the number of fins 16 is not limited to four but can be any other number. Moreover, while the fins 16 in the shown anchoring member 50 are essentially equally sized, other embodiments include differently sized fins, like for example a configuration in which one pair of opposing fins is smaller than another pair of opposing fins in the direction of the insertion or the direction radial thereto.

In comparison to the anchoring member 1 shown in Fig. 1 , the embodiment shown in Fig. 2 is only inserted into the cranial portion of the humerus bone.

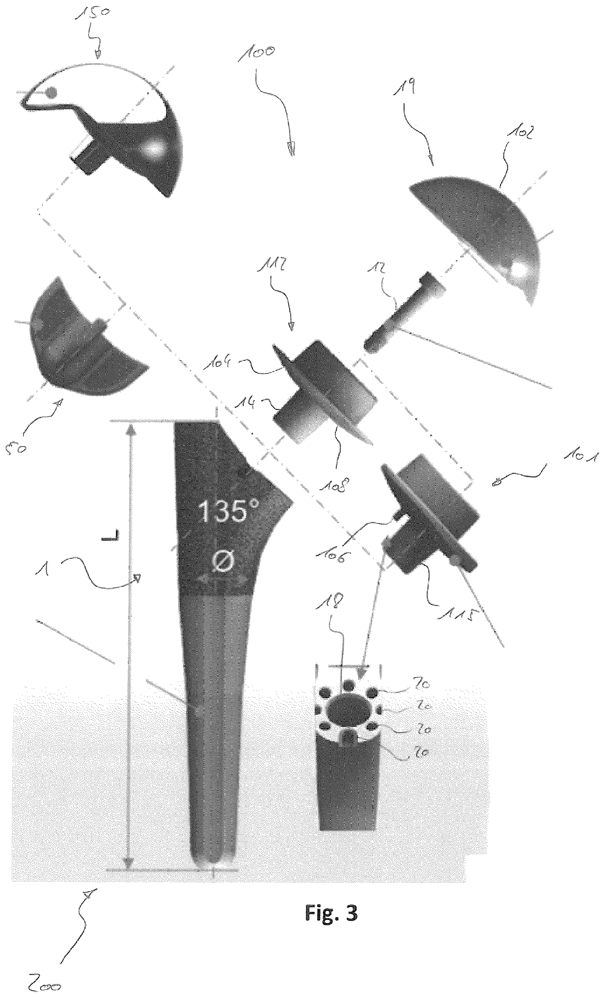

Fig. 3 shows, in a side view, an arrangement of a joint replacement component 200.

The joint replacement component 200 comprises an anchoring member, which can be essentially one of the anchoring members 1, 50 described above, and a joint member 100. In the example shown in Fig. 3 , the joint member 100 is an anatomical joint member. However, the joint member 100 may also be a reverse joint member.

In the example shown in Fig. 3 , the joint member 100 comprises a joint component 19 with a proximal end 102, which is formed as a joint surface, and an intermediate part 112 comprising a distal interface part 104 with a convex interface surface 108. The distal interface part 104 comprises a convex interface surface 108 and is configured to be mounted to an anchoring member 1, 50 of the joint replacement. The joint surface of the proximal end 102 is formed as a convex ball joint surface. It is to be noted that, however, the proximal end 102 can also be formed as a concave ball joint surface in the case of a reverse joint replacement.

As shown in the example of Fig. 3 , the joint member 100 may be composed of separate parts, such as the intermediate component 112 and the joint component 19 The intermediate component 112 may be fixed to the anchoring member 1, 50 by means of for example fixation member 12, while the joint component 19 may be fixed to the intermediate component 112 by means of a friction fit or other methods known in the art.

Anchoring member 1, 50 can also be connected to an intermediate component 101 with an offset fixation member 115 and an indexing structure 106.

It is, however, to be noted that the anchoring members 1, 50 may also be connected to an integrally formed joint member 150.

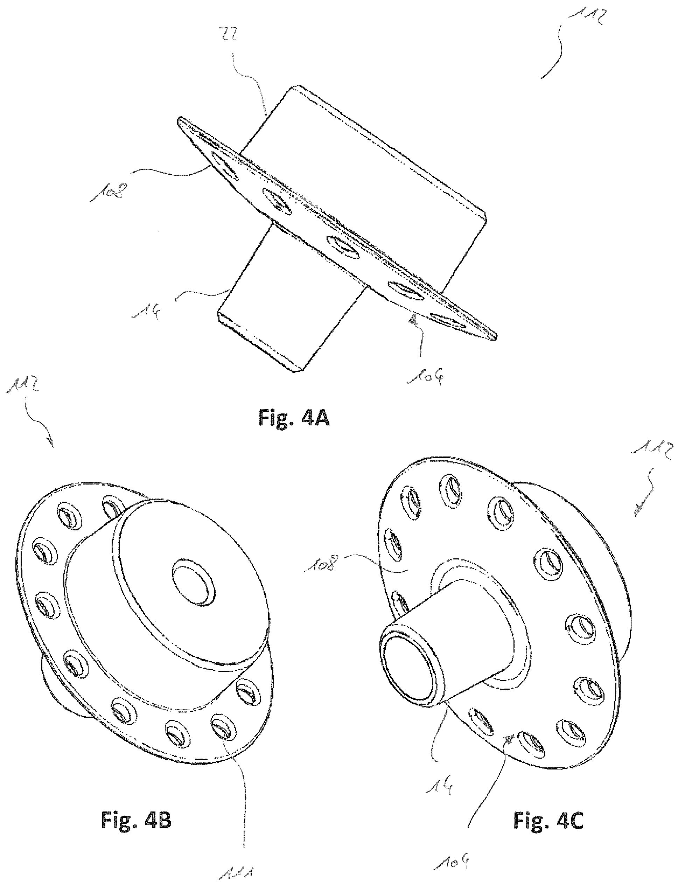

Figs. 4A, 4B, and 4C show different views of an example of the intermediate component 112. The intermediate component 112 comprises a proximal engagement portion 22, which is configured to be engaged with the joint component 19 (not shown in Figs. 4 ), and a distal interface part 104, which comprises a convex interface surface 108. The distal interface part 104 further comprises a fixation member 14, which in this case is a protrusion that is configured to mate with a corresponding mounting hole 18 of an anchoring member 1, 50 (see description above). That is, the fixation member 14 can have a thread and/or can be tapered in order to engage with the mounting hole 18 by means of a friction fit. Additionally or alternatively, it may be in engagement via a form fit.

The intermediate component 112 shown in Figs. 4A, 4B, 4C also comprises a plurality of holes 111 arranged circumferentially and penetrating the distal interface part, which can for example be used as suture holes.

In case that the intermediate component 112 is connected to the anchoring member 1, 50 by means of a separate fixation member 12, such as a screw or a rivet, the fixation member 14 may be formed so as to center the intermediate component 112 with respect to the mounting hole 18.

In the example shown in Fig. 4 the fixation member 14 is provided centered on the interface surface 108 of the intermediate component 112. However, it is to be noted that the fixation member 14 can also be disposed offset from the rotational center of the distal interface part 104 such that a rotation about the fixation member can be used to adjust the orientation of the intermediate component 112 with respect to, for example, the mounting hole 18 of an anchoring member 1, 50.

In addition, there may be provided an indexing structure. More specifically, a protrusion (not shown) next to the fixation member 14 may engage a corresponding indexing recess 20 of an anchoring member 1, 50.

Preferably, the convex interface surface 108 has a curvature, in particular a radius, that is essentially identical to or smaller than the curvature, in particular radius, of the concave interface surface 7 of an anchoring member 1, 50 to be engaged with the intermediate component 112. In case that the fixation member 14 is configured to form a tapered connection with the mounting hole 18 of the anchoring member 1, 50, the convex interface surface 108 of the intermediate member 112 and the concave interface surface 7 of the anchoring member come close to each other, but do not contact each other, such that the intermediate member 112 can be placed to some extent inside the anchoring member 1, 50, which allows to reduce the space required for the joint replacement component 200. More specifically, the interface surfaces 7, 108 are preferably at a distance to each other such as a distance smaller than 3, 2, or 1 mm. This distance may (also) be caused by the curvature of the convex interface surface 108 being smaller than the curvature of the concave interface surface 7.

In case that the fixation member 14 is configured to be in a threaded engagement with the mounting hole 18 of the anchoring member 1, 50, the interface surfaces of the anchoring member 1, 50 and the intermediate part 112 may be in contact with each other. Also in this case, the convex and concave shape of the intermediate part 112 and the anchoring member 1, 50, respectively, allow for a reduced volume for the joint replacement component 200.

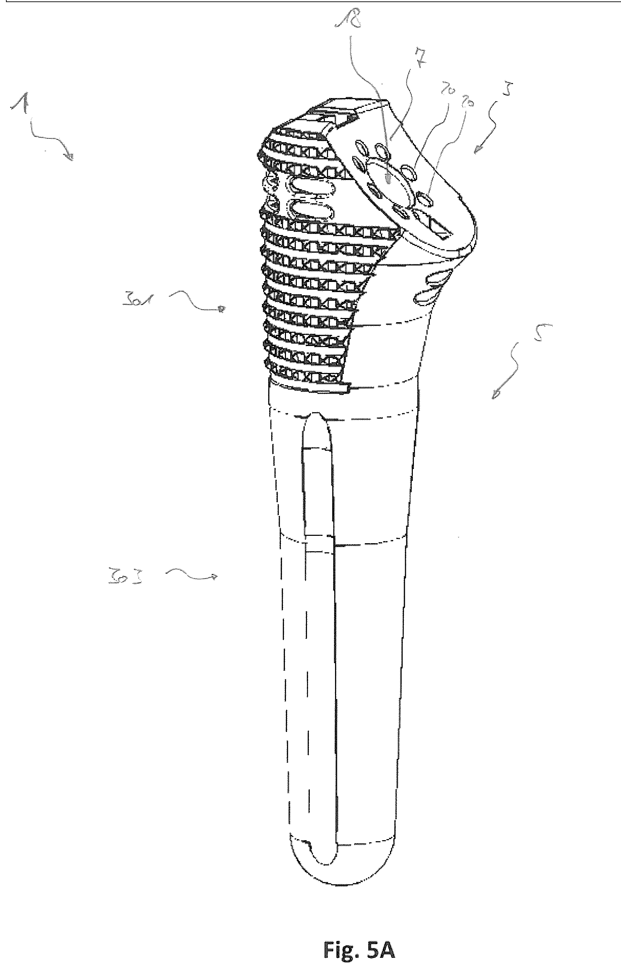

Fig. 5A shows an embodiment of a modular anchoring portion 1. In the embodiment shown in Fig. 5A , the anchoring member 1 comprises a proximal interface part 3 and a distal portion 5. The proximal interface part 3 of the embodiment shown in Fig. 5A comprises a concave interface surface 7, preferably formed as a recess, and a central mounting hole 18. Further, the proximal interface part 3 comprises indexing recesses 20 arranged circularly around the mounting hole 18.

The distal portion 5 of the anchoring member 1 shown in Fig. 5A is configured to be anchored within bone tissue and has a modular configuration. In particular, the modular anchoring portion 1 comprises a proximal part 301, and a distal part 303, which are attached to each other, for example by means of a fastener (not shown).

The distal part 303 of the embodiment may have a substantially even surface for being cemented or a surface configured for bone ingrowth. The proximal part 301 may have a textured surface for supporting the attachment of soft tissue structures.

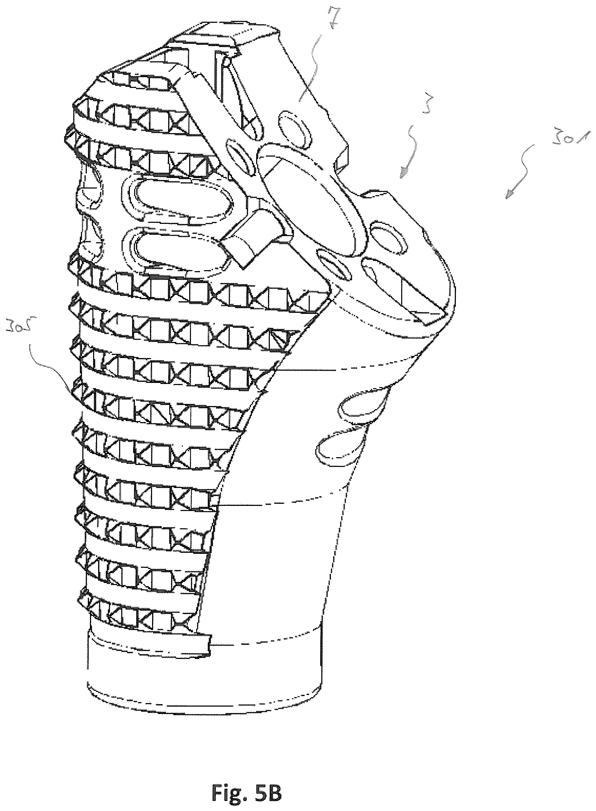

Fig. 5B shows an embodiment of a proximal part 301. In the embodiment shown in Fig. 5B , the surface of proximal part 301 is textured by protrusions 305, which protrude from a part of the outer surface of the proximal part 305. Said protrusions 305 enhance the interaction with surrounding tissue, for example with bone tissue or with soft tissue, and enhances the stability of the anchoring member.

Proximal part 301 and distal part 303 may be made of different materials.

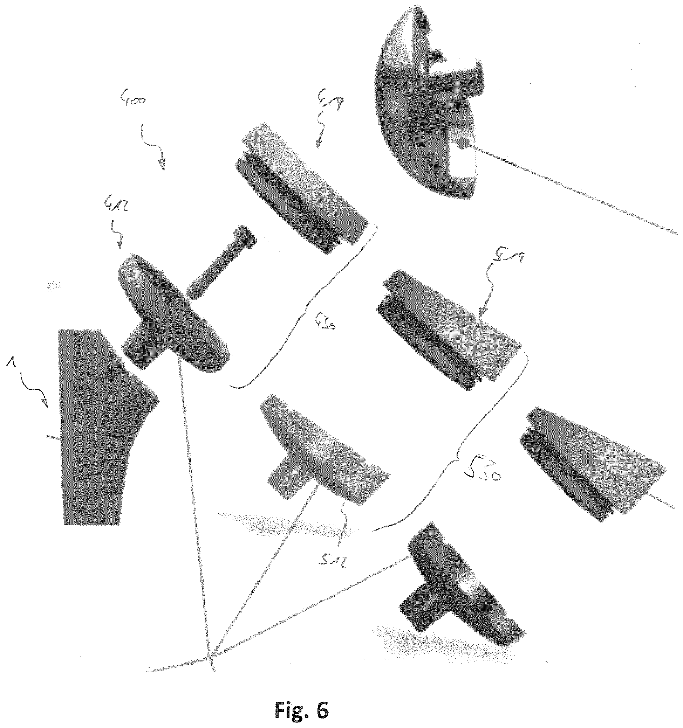

Fig. 6 shows exploded views of different examples of a reverse joint replacement.

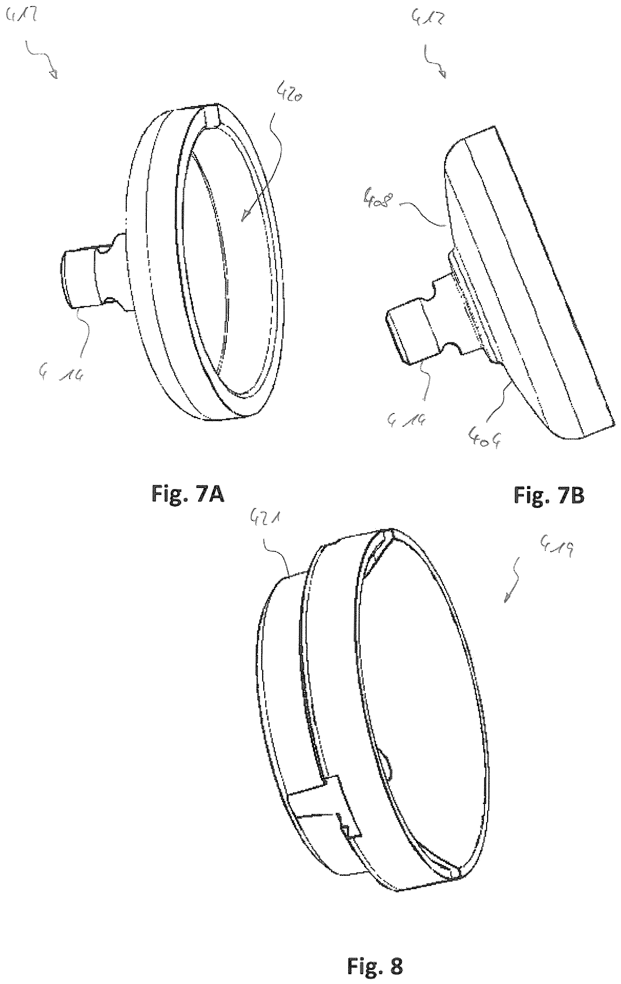

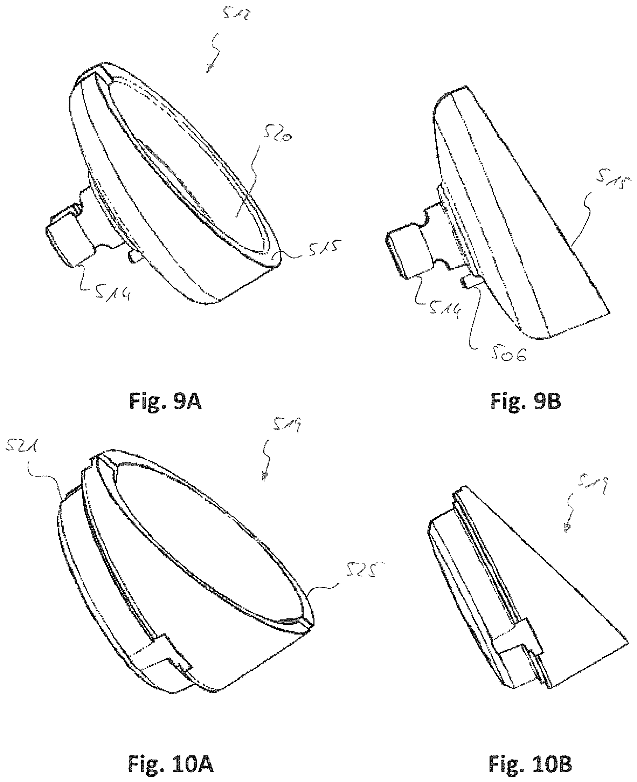

As illustrated, an anchoring member 1, 50, can be connected to an intermediate component 412, which forms part of a reverse joint member 430 for a reverse joint replacement. For example, the anchoring member 1, 50 can be connected to an intermediate component 412 shown in Fig. 7A and Fig. 7B , which, in turn, can be connected to a reverse joint component 419 shown in Fig. 8 . Alternatively, anchoring member 1, 50 can be connected to an inclined intermediate component 512 shown in Fig. 9A and Fig. 9B , which, in turn, can be connected to an inclined reverse joint component 519 shown in Figs. 10A and 10B .

Intermediate component 412 has, similar to intermediate component 112, a distal interface part 404 with a convex interface surface 408, on which a fixation member 414 is positioned.

Intermediate component 412 has a recess 420, which can be configured with a thread or taper in order to fixedly engage with a corresponding protrusion 421 of a joint component 419 for a reverse joint replacement.

Alternatively, a reverse joint member 530 can be an assembly of an intermediate component 512, as shown in Figures 9A and 9B , and a joint component 519, as shown in Figures 10A and 10B .

For a reverse joint replacement, an intermediate component 512 is formed with a recess 520 for engaging with a corresponding protrusion 521 of a joint component 519. The intermediate component 512 can be formed with an inclined rim 515 or mounting plane with respect to the axis of the fixation member 514.

Additionally, intermediate component 512 is provided with an indexing protrusion 506, which is configured so as to be engageable with the indexing recesses 20 of an anchoring member 1, 50 when the fixation member 14 is in engagement with the mounting hole 18 of the anchoring member 1, 50. This allows for adjusting the direction of inclination of the plane of the intermediate member 512 with respect to the anchoring member 1, 50.

The joint component 519 shown in Figs. 10A and 10B is configured with a protrusion 521 to engage with the corresponding recess 520 of the intermediate component 512. The joint component 519 has a proximal end 502 that is formed as a concave joint surface in order to interact with a corresponding convex glenoidal joint member (not shown).

The joint surface has a rim 525 which is inclined with respect to the axis of the protrusion 521, such that the joint component 519 defines an inclined joint surface.

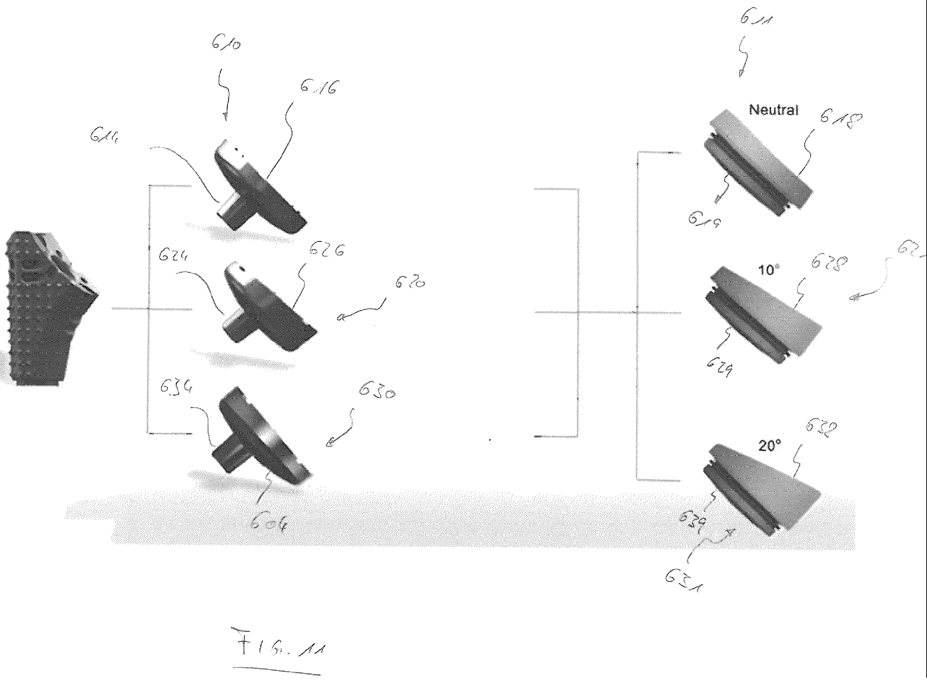

The inclination of the joint surface as provided by the inclined intermediate component 512 and the inclined joint component 519 can be selected in accordance with the patient's anatomy as shown in Fig. 11 .

In this respect, Figure 11 shows:
- a neutral intermediate component 610, in which the central axis of the fixation member 614 and the axis of the plane 616 are substantially aligned;
- a positively inclined intermediate component 620, in which the axis of the plane 626 deviates in a cranial direction from the axis of the fixation member 624; and
- a neutral intermediate component 630, in which the central axis of the fixation member 634 and the central axis of the distal interface part 604 are offset.

Further, Figure 11 illustrates:
- neutral joint component 611, in which the cranial plane 618 and the caudal plane 619 are substantially parallel,
- a 10° inclined joint component 621, in which the cranial plane 628 and the caudal plane 629 form a 10° angle, and
- a 20° inclined joint component 631, in which the cranial plane 638 and the caudal plane 639 form a 20° angle.

The intermediate components 610, 620, and 630 and the joint components 611, 621, and 631 can be assembled as required for the respective patient.

Different inclinations and offsets of the intermediate component 610, 620, 630, and joint component 611, 621, 631 allow the surgeon to adapt the configuration to a specific case.

In the examples illustrated in figures 6 to 11 both the intermediate components and the joint components are provided in different inclinations. As previously explained, it is therefore possible to adjust the orientation of the articular joint by choosing the inclined intermediate components 610, 620, 630, and joint components 611, 621, 631. Further, the inclination to the anchoring member or angle between the longitudinal axis of the anchoring member central axis of the joint surface may be adjusted at the level of the intermediate component and/or the level of the joint component. For example, the plane defined by the rim 515 of the intermediate component 512 may be inclined in relation to the interface surface 7 of the anchoring member 1 ( Figs. 9 ) and/or the plane defined by the rim 525 of the joint component 519 may be inclined in relation to the rim 515 of the intermediate component 512.

It is to be noted that the examples shown in Figures 7A, 7B , 9A, and 9B show exemplary arrangements using snap-fit connections as fixation member 414 and 514, while the examples shown in Fig. 11 have taper connections 614, 624, 634. The former may preferably be used as trial components whereas the latter may preferably be used as functional components, i. e. components that remain implanted.

### LIST OF REFERENCE SIGNS

- 1: Anchoring member
- 3: Proximal interface part
- 5: Distal portion
- 7: Interface surface
- 12: Fixation member
- 14: Fixation member
- 15: Stem portion
- 16: Fin
- 18: Central hole
- 19: Joint component
- 20: Indexing structure
- 22: Proximal engagement portion
- 22: Proximal engagement portion
- 50: Anchoring member
- 100: Joint member
- 101: Joint member with offset fixation member
- 102: Proximal End
- 104: Distal interface part
- 106: Indexing protrusion
- 108: Convex interface surface
- 111: Suture holes
- 112: Intermediate part
- 115: Fixation member
- 150: Joint member
- 301: Proximal part
- 302: Distal part
- 305: Protrusion
- 400: Joint replacement component
- 430, 530: Joint member
- 412, 512: Intermediate component
- 419, 519: Joint component
- 414, 514: Fixation member
- 420, 520: Mounting recess
- 421, 521: Protrusion
- 515: Rim of intermediate component
- 525: Rim of joint component
- 506: Indexing protrusion
- 604: Fixation member
- 610, 620, 630: Intermediate component
- 611, 621, 631: Joint component
- 614, 624, 634: Fixation member
- 616, 626: Plane
- 618, 628, 638: Cranial plane
- 619, 629, 639: Caudal plane

## Claims

1. An anchoring member (1) for a joint replacement,
the anchoring member (1) comprising a proximal interface part (3) and a distal portion (5), the distal portion (5) being configured to be anchored within bone tissue and the proximal interface part (3) being configured for mounting a joint member (100) of the joint replacement,
wherein the proximal interface part (3) comprises a concave interface surface (7),
**characterized in that** the concave interface surface (7) comprises an indexing structure.

2. The anchoring member (1) according to claim 1, wherein the concave interface surface (7) is formed as a recess.

3. The anchoring member (1) according to claim 2,
wherein the concave recess has a curved profile, the curved profile being spherical, ellipsoidal, or ovoid shaped.

4. The anchoring member (1) according to any one of the preceding claims,
wherein the anchoring member (1) comprises a mounting hole (18) in the proximal interface part (3) for fixedly attaching a joint member (100), the mounting hole preferably comprising a thread for engagement with a fixation member (12, 14) and/or a tapered portion.

5. The anchoring member (1) according to any one of the preceding claims,
wherein the anchoring member (1) comprises a plurality of fins (16) extending in radial directions in relation to a direction of insertion of the anchoring member (1) into the bone.

6. The anchoring member (1) according to claim 5,
wherein the plurality of fins (16) comprises two pairs of two essentially symmetrical fins (16) arranged opposite to each other with respect to the direction of insertion of the anchoring member (1) into the bone.

7. The anchoring member according to any one of the preceding claims,
wherein the indexing structure comprises one or more indexing recesses (20) for mating each with a corresponding protrusion (106) of the joint member (100).

8. The anchoring member according to claim 7,
wherein the indexing recesses (20) are arranged equidistantly on a circle around the central axis of the mounting hole (18).

9. The anchoring member (1) according to any of the preceding claims,
the anchoring member (1) further comprising a stem part (15) that is configured to be inserted into the bone, wherein the stem part (15) is preferably modular.

10. A joint member (100) for a joint replacement,
the joint member comprising a proximal end (102) and a distal interface part (104), the proximal end (102) being formed as a joint surface and the distal interface part (104) being configured for mounting an anchoring member (1) according to any of claims 1 to 9, wherein the interface surface (108) of the joint member (1) includes an indexing structure,
wherein the joint surface is a ball joint surface, and
wherein the distal interface part (104) comprises a convex interface surface.

11. The joint member (100) according to claim 10,
wherein the joint member (100) comprises an intermediate component (112) and a joint component,
wherein the intermediate component (112) and the joint component are preferably fixedly attached to each other by means of a friction fit or a form fit.

12. The joint member (100) according to claim 10 or 11, wherein the indexing structure is a protrusion (106) configured so as to mate with an indexing recess of an anchoring member.

13. The joint member (100) according to any one of claims 10 to 12, wherein the joint member is configured as an anatomical or reverse joint member.

14. A joint replacement component (200), the joint replacement component being for replacing one side of a joint, the joint replacement component comprising:
an anchoring member (1) according to any one of claims 1 to 9, and
a joint member (100) according to any one of claims 10 to 14,
wherein, in an assembled state, the concave interface surface (7) of the anchoring member and the convex interface surface (108) of the joint member (100) are in surface contact so that the joint member is partly accommodated within the anchoring member.

15. The joint replacement component (200) according to claim 14,
wherein the joint replacement is a replacement for a spherical joint, in particular for a shoulder joint.

## Patentansprüche

1. Verankerungselement (1) für einen Gelenkersatz,
wobei das Verankerungselement (1) ein proximales Grenzflächenteil (3) und einen distalen Abschnitt (5) umfasst, wobei der distale Abschnitt (5) dazu konfiguriert ist, innerhalb von Knochengewebe verankert zu sein, und das proximale Grenzflächenteil (3) dazu konfiguriert ist, ein Gelenkelement (100) des Gelenkersatzes zu befestigen,
wobei das proximale Grenzflächenteil (3) eine konkave Grenzflächenoberfläche (7) umfasst,
**dadurch gekennzeichnet, dass** die konkave Grenzflächenoberfläche (7) eine Indexierungskonstruktion aufweist.

2. Verankerungselement (1) nach Anspruch 1,
wobei die konkave Grenzflächenoberfläche (7) als Vertiefung ausgebildet ist.

3. Verankerungselement (1) nach Anspruch 2,
wobei die konkave Vertiefung ein gekrümmtes Profil aufweist,
wobei das gekrümmte Profil kugelförmig, ellipsoid oder eiförmig ist.

4. Verankerungselement (1) nach einem der vorhergehenden Ansprüche,
wobei das Verankerungselement (1) ein Befestigungsloch (18) in dem proximalen Grenzflächenteil (3) zum festen Anbringen eines Gelenkelements (100) umfasst, wobei das Montageloch vorzugsweise ein Gewinde zum Eingriff mit einem Fixierungselement (12, 14) und/oder einem konischen Abschnitt umfasst.

5. Verankerungselement (1) nach einem der vorhergehenden Ansprüche,
wobei das Verankerungselement (1) eine Vielzahl von Rippen (16) umfasst, die sich im Hinblick auf eine Einsetzrichtung des Verankerungselements (1) in den Knochen in radialen Richtungen erstreckt.

6. Verankerungselement (1) nach Anspruch 5,
wobei die Vielzahl von Rippen (16) zwei Paare von zwei im Wesentlichen symmetrischen Rippen (16) umfasst, die in Bezug auf die Einsetzrichtung des Verankerungselements (1) in den Knochen einander gegenüberliegend angeordnet sind.

7. Verankerungselement nach einem der vorhergehenden Ansprüche, wobei die Indexierungskonstruktion eine oder mehrere Indexierungsvertiefungen (20) umfasst, die jeweils mit einem entsprechenden Vorsprung (106) des Gelenkelements (100) zusammenpassen.

8. Verankerungselement nach Anspruch 7,
wobei die Indexierungsvertiefungen (20) in gleichem Abstand auf einem Kreis um die Mittelachse des Befestigungslochs (18) herum angeordnet sind.

9. Verankerungselement (1) nach einem der vorhergehenden Ansprüche,
wobei das Verankerungselement (1) ferner ein Schaftteil (15) umfasst, das dazu konfiguriert ist, in den Knochen eingesetzt zu sein, wobei das Schaftteil (15) vorzugsweise modular ist.

10. Gelenkelement (100) für einen Gelenkersatz,
wobei das Gelenkelement ein proximales Ende (102) und ein distales Grenzflächenteil (104) umfasst, wobei das proximale Ende (102) als eine Gelenkfläche ausgebildet ist und das distale Grenzflächenteil (104) zum Befestigen eines Verankerungselements (1) nach einem der Ansprüche 1 bis 9 konfiguriert ist, wobei die Grenzflächenoberfläche (108) des Gelenkelements (1) eine Indexierungskonstruktion beinhaltet, wobei die Gelenkfläche eine Kugelgelenkfläche ist und
wobei das distale Grenzflächenteil (104) eine konkave Grenzflächenoberfläche umfasst.

11. Gelenkelement (100) nach Anspruch 10,
wobei das Gelenkelement (100) eine Zwischenkomponente (112) und eine Gelenkkomponente umfasst,
wobei die Zwischenkomponente (112) und die Gelenkkomponente vorzugsweise durch Reibschluss oder Formschluss fest aneinander angebracht sind.

12. Gelenkelement (100) nach Anspruch 10 oder 11,
wobei die Indexierungskonstruktion ein Vorsprung (106) ist, der dazu konfiguriert ist, mit einer Indexierungsvertiefung eines Verankerungselements zusammenzupassen.

13. Gelenkelement (100) nach einem der Ansprüche 10 bis 12, wobei das Gelenkelement als anatomisches oder umgekehrtes Gelenkelement konfiguriert ist.

14. Gelenkersatzkomponente (200), wobei die Gelenkersatzkomponente zum Ersetzen einer Seite eines Gelenks dient, wobei die Gelenkersatzkomponente Folgendes umfasst:
ein Verankerungselement (1) nach einem der Ansprüche 1 bis 9 und ein Gelenkelement (100) nach einem der Ansprüche 10 bis 14,
wobei in einem zusammengebauten Zustand die konkave Grenzflächenoberfläche (7) des Verankerungselements und die konvexe Grenzflächenoberfläche (108) des Gelenkelements (100) in Flächenkontakt stehen, sodass das Gelenkelement teilweise innerhalb des Verankerungselements aufgenommen ist.

15. Gelenkersatzkomponente (200) nach Anspruch 14,
wobei der Gelenkersatz ein Ersatz für ein Kugelgelenk,
insbesondere für ein Schultergelenk, ist.

## Revendications

1. Élément d'ancrage (1) de remplacement d'articulation, l'élément d'ancrage (1) comprenant une partie d'interface proximale (3) et une partie distale (5), la partie distale (5) étant configurée pour être ancrée dans le tissu osseux et la partie d'interface proximale (3) étant configurée pour le montage d'un élément d'articulation (100) du remplacement d'articulation,
dans lequel la partie d'interface proximale (3) comprend une surface d'interface concave (7),
**caractérisé en ce que** la surface d'interface concave (7) comprend une structure d'indexation.

2. Élément d'ancrage (1) selon la revendication 1,
dans lequel la surface d'interface concave (7) est formée comme un évidement.

3. Élément d'ancrage (1) selon la revendication 2,
dans lequel l'évidement concave a un profil incurvé, le profil incurvé étant de forme sphérique, ellipsoïdale ou ovoïde.

4. Élément d'ancrage (1) selon l'une quelconque des revendications précédentes,
dans lequel l'élément d'ancrage (1) comprend un trou de montage (18) dans la partie d'interface proximale (3) pour fixer de manière fixe un élément d'articulation (100), le trou de montage comprenant de préférence un filetage pour venir en prise avec un élément de fixation (12, 14) et/ou une partie conique.

5. Élément d'ancrage (1) selon l'une quelconque des revendications précédentes,
dans lequel l'élément d'ancrage (1) comprend une pluralité d'ailettes (16) s'étendant dans des directions radiales par rapport à une direction d'insertion de l'élément d'ancrage (1) dans l'os.

6. Élément d'ancrage (1) selon la revendication 5,
dans lequel la pluralité d'ailettes (16) comprend deux paires de deux ailettes essentiellement symétriques (16) disposées en regard l'une de l'autre par rapport à la direction d'insertion de l'élément d'ancrage (1) dans l'os.

7. Élément d'ancrage selon l'une quelconque des revendications précédentes,
dans lequel la structure d'indexation comprend un ou plusieurs évidements d'indexation (20) destinés à s'accoupler chacun avec une saillie correspondante (106) de l'élément d'articulation (100).

8. Élément d'ancrage selon la revendication 7,
dans lequel les évidements d'indexation (20) sont disposés de manière équidistante sur un cercle autour de l'axe central du trou de montage (18).

9. Élément d'ancrage (1) selon l'une quelconque des revendications précédentes,
l'élément d'ancrage (1) comprenant également une partie tige (15) qui est configurée pour être insérée dans l'os, dans lequel la partie tige (15) est de préférence modulaire.

10. Élément d'articulation (100) pour un remplacement d'articulation,
l'élément d'articulation comprenant une extrémité proximale (102) et une partie d'interface distale (104), l'extrémité proximale (102) étant formée comme une surface d'articulation et la partie d'interface distale (104) étant configurée pour le montage d'un élément d'ancrage (1) selon l'une quelconque des revendications 1 à 9, dans lequel la surface d'interface (108) de l'élément d'articulation (1) comporte une structure d'indexation,
dans lequel la surface d'articulation est une surface d'articulation à rotule, et
dans lequel la partie d'interface distale (104) comprend une surface d'interface convexe.

11. Élément d'articulation (100) selon la revendication 10, dans lequel l'élément d'articulation (100) comprend un composant intermédiaire (112) et un composant d'articulation,
dans lequel le composant intermédiaire (112) et le composant d'articulation sont de préférence fixés de manière fixe l'un à l'autre au moyen d'un ajustement par friction ou d'un ajustement par forme.

12. Élément d'articulation (100) selon la revendication 10 ou 11,
dans lequel la structure d'indexation est une saillie (106) configurée de manière à s'accoupler avec un évidement d'indexation d'un élément d'ancrage.

13. Élément d'articulation (100) selon l'une quelconque des revendications 10 à 12, dans lequel l'élément d'articulation est configuré comme un élément d'articulation anatomique ou inversé.

14. Composant de remplacement d'articulation (200), le composant de remplacement d'articulation étant destiné à remplacer un côté d'une articulation, le composant de remplacement d'articulation comprenant :
un élément d'ancrage (1) selon l'une quelconque des revendications 1 à 9, et
un élément d'articulation (100) selon l'une quelconque des revendications 10 à 14,
dans lequel, dans un état assemblé, la surface d'interface concave (7) de l'élément d'ancrage et la surface d'interface convexe (108) de l'élément d'articulation (100) sont en contact de surface de sorte que l'élément d'articulation est partiellement logé à l'intérieur de l'élément d'ancrage.

15. Composant de remplacement d'articulation (200) selon la revendication 14,
dans lequel le remplacement d'articulation est un remplacement d'une articulation sphérique, en particulier d'une articulation d'épaule.
